Europäisches Patentamt

European Patent Office

Office européen des brevets

(19)

(11) Publication number: **0 027 216**
**A1**

## (12) EUROPEAN PATENT APPLICATION

(21) Application number: **80105999.9**

(22) Date of filing: **03.10.80**

(51) Int. Cl.³: **C 07 J 9/00**

(30) Priority: **04.10.79 US 81954**

(43) Date of publication of application:
**22.04.81 Bulletin 81/16**

(84) Designated Contracting States:
**BE CH DE FR GB LI NL**

(71) Applicant: **EASTMAN KODAK COMPANY**
**343 State Street**
**Rochester, New York 14650(US)**

(72) Inventor: **Nelan, Donald Royce**

**Deceased(US)**

(74) Representative: **Brandes, Jürgen, Dr.**
**Dipl.-Chem. et al,**
**Patentanwälte H. Bartels, Dipl.-Chem. Dr. Brandes**
**Dr.-Ing. Held, Dipl.-Phys. Wolff Thierschstrasse 8**
**D-8000 München 22(DE)**

(54) **Process for dehydrogenation of beta-hydroxy steroids.**

(57) This invention relates to the dehydrogenation of $3$-$\beta$-hydroxy steroids, such as a mixture of soy sterols, to form the corresponding $\Delta^4$-3-keto derivatives of the steroids. Copper or palladium is used as the catalyst and a dialkyl ketone is used as a solvent.

EP 0 027 216 A1

-1-

## PROCESS FOR DEHYDROGENATION OF 3-β-HYDROXY STEROIDS

This invention relates to the dehydrogenation of 3-β-hydroxy steroids, such as a mixture of soy sterols to form the corresponding $\Delta^4$-3-keto derivatives of the steroids using as a catalyst either copper or palladium and using as the solvent a dialkyl ketone.

The naturally occurring phytosterol components of vegetable oils, such as soy oils are composed of mixtures of phytosterols. These phytosterols can be used in the preparation of pharmaceuticals, such as progesterone, which also can be used to prepare other steroids, such as cortisone. The first step in the preparation of progesterone from soy sterols involves the dehydrogenation of the sterols to form the $\Delta^4$-3-keto derivatives. The 3β-OH of the sterol is dehydrogenated to a ketone with rearrangement of the $\Delta^5$ double bond into conjugation with the ketone. Catalytic dehydrogenation is an economical method for this oxidation-rearrangement process. Catalytic dehydrogenation provides a process for dehydrogenation of $\Delta^5$-sterols to ketones with a simultaneous shift of the $\Delta^5$ double bond to the $\Delta^4$ position in conjugation with the carbonyl group. Raney nickel has been used for this reaction, as disclosed in Chakravarti, Chakravarti, and Mitra, Nature, 193, 1071 (1962) or in the presence of a hydrogen acceptor as disclosed in E. C. Kleiderer, and E. C. Kornfeld, J. Org. Chem., 13, 455 (1948) and Kleiderer, Rice, Conquest and Williams, U.S. Dept. of Commerce, Office of the Publication Board, Report PB 981, 1945.

The reaction disclosed by Kleiderer et al was carried out with Raney nickel as a catalyst and cyclohexanone as a hydrogen acceptor. The reaction was carried out for a period of 24 hours and the yield of product varied from 30 to 80 percent. After completion of the reaction catalyst was filtered from the reaction mixture and solvent was removed by distillation. Further treatment of the residue with ether, filtering and recrystallization with ethanol were required to obtain the desired reaction product.

In accordance with this invention product yields at least as high as the prior art can be obtained and the further treatment of the residue can be eliminated. The product can be obtained by the simple procedure of filtration of the catalyst and evaporation of solvent. In this invention 3-$\beta$-hydroxy steroids, such as mixtures of soy sterols, are dehydrogenated to form the corresponding $\Delta^4$-3-keto derivatives using copper or palladium as a catalyst and a dialkyl ketone as a solvent.

The dehydrogenation can be carried out over periods of time from 8 to 30 hours, preferably 10-24 hours and most preferably 14-18 hours. The temperature at which the dehydrogenation is carried out is from 200-350°C., preferably 250-325°C. and most preferably 275-300°C. At temperatures lower than 200°C., the rate of dehydrogenation is too slow for effective reaction. At temperatures greater than 350°C. dehydration occurs with some decomposition of the steroids. The dialkyl ketone solvent can be any lower alkyl ketone, such as dimethyl ketone, diethyl ketone, dipropyl ketone, dibutyl ketone, methyl ethyl ketone and methyl butyl ketone. The amount of solvent used depends on whether the process of the present invention is to be operated as a batch process or a continuous process. A greater amount of solvent is generally needed to operate a continuous process, however, an amount of solvent five times the amount of steroids employed is generally satisfactory for both types of processes.

The catalyst preferably is a particulate metallic material having a large surface area. Forms of such catalyst therefore can be, for example, in the form of dust or other large surface area forms of copper such as copper wire or gauze. The palladium should likewise have a large surface area and should be in the form of sponge or other similar large surface area forms of palladium. The amount of catalyst employed varies with the amount of steroids used and the speed of reaction desired for the dehydrogenation reaction. Generally, an amount of catalyst used can be equal to about 20 to 40 percent, preferably 25 to 30 percent, based on the weight of the steroids to be dehydrogenated.

EXAMPLE 1

To 300 g. of mixed soy sterols (~20% stigmasterol) in 1500 ml of methyl ethyl ketone was added 100 g. of copper wire. After heating at 275°C. 16 hrs. in a rocking autoclave, the $\Delta^4$-3-keto derivatives of soy sterols were isolated by filtering the catalyst and evaporation of the solvent from the filtrate (isolated 291 g.). The $\Delta^4$-3-keto derivatives of soy sterols are then reacted by ozonolysis to form 3-ketodinor-4-chclen-22-aldehyde which can be isolated from the other $\Delta^4$-3-keto derivatives of soy sterols by either chromatography or by treatment with sodium bisulfite and extraction with a suitable organic solvent such as toluene.

EXAMPLE 2

To 200 g. of mixed soy sterols (~20% stigmasterol) in 1500 ml of methyl ethyl ketone was added 50 g. of palladium sponge. After heating at 300°C. 8 hrs. in a rocking autoclave, the $\Delta^4$-3-keto derivatives of soy sterols were isolated by filtering the catalyst and evaporation of the solvent from the filtrate to obtain 205 g. of $\Delta^4$-3-keto derivatives of soy sterols.

EXAMPLE 3

Cholesterol (24 g), methyl ethyl ketone (150 ml), and copper (6.5 g) were heated in a rocking autoclave at 275°C. for 8 hrs. An analysis by gas chromatography indicated 60% unreacted cholesterol and 30% 4-cholesten-3-one. The mixture was reheated in the rocking autoclave at 275°C. for 8 more hours and then the catalyst was filtered and the solvent evaporated in vacuo from the filtrate to give 23 g. solids analyzed by gas chromatography to show 11% cholesterol and 75% 4-cholesten-3-one. Approximately 90% of the cholesterol was converted to 4-cholesten-3-one in 83% yield.

EXAMPLE 4

Cholesterol (20 g), methyl ethyl ketone (150 ml), and palladium sponge (5.0 g) were heated in a rocking autoclave at 300°C. for 16 hr. Analysis of an aliquot of the reaction solution by gas chromatography showed 9% cholesterol and 91% 4-cholesten-3-one. The catalyst was filtered and the filtrate was concentrated in vacuo to give 20.5 g white solids. Approximately 90% of the cholesterol was converted to 4-cholesten-3-one in the presence of palladium. Similar results can be obtained if cholesterol is replaced with sitosterol.

The process of the present invention provides an improved method for the dehydrogenation of 3-β-hydroxy steroids to provide $\Delta^4$-3-ketosteroids. These 4-en-3-ones derivatives can be used to provide materials useful for preparation of valuable steroids such as the cortical steroids.

Claims:

1. A process for dehydrogenating 3-β-hydroxy steroids to form the corresponding $\Delta^4$-3-keto derivatives characterized by using copper or palladium as a catalyst and a dialkyl ketone as a solvent.

2. A process according to Claim 1 wherein the catalyst is copper.

3. A process according to Claim 1 wherein the catalyst is palladium.

4. A process according to Claim 1 wherein methyl ethyl ketone is the solvent.

0027216

European Patent
Office

EUROPEAN SEARCH REPORT

Application number

EP 80 10 5999

| Category | Citation of document with indication, where appropriate, of relevant passages | Relevant to claim |
|---|---|---|
| D,A | JOURNAL OF ORGANIC CHEMISTRY, vol. XIII, no. 3, May 1948, pages 455-458 <br> Washington D.C., U.S.A. <br> E.C. KLEIDERER et al.: "Raney nickel as an organic oxidation-reduction catalyst" <br><br> * Whole article * | 1 |
| A | CHEMICAL ABSTRACTS, vol. 48, no. 9, 14th May 1954, page 5202, column 2, no. 5202e <br> Columbus, Ohio, U.S.A. <br> J. ROMO: "Catalytic oxidations with Raney nickel" <br><br> & BOL. INST. QUIM. UNIV. NACL. AUTON. MEX. 4, 91-100, 1952 <br><br> * Abstract * | 1 |
| A | US - A - 2 890 226 (ARTHUR R. HANZE) <br><br> * Whole patent * | 1 |
| A | RECUEIL DES TRAVAUX CHIMIQUES DES PAYS BAS, vol. 56, no. 1, January 1937, pages 137-144 <br> Amsterdam, NL. <br> R.V. OPPENAUER: "Eine Methode der Dehydrierung von sekundaren Alkoholen zu Ketonen. I. Zur Herstellung von Sterinketonen und Sexualhormonen" <br><br> * Whole article * | 1 |

**DOCUMENTS CONSIDERED TO BE RELEVANT**

**CLASSIFICATION OF THE APPLICATION (Int. Cl.³)**

C 07 J 9/00

**TECHNICAL FIELDS SEARCHED (Int. Cl.³)**

C 07 J 9/00

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 11-12-1980 | HENRY |

EPO Form 1503.1  06.78